# EUROPÄISCHE PATENTANMELDUNG

(11) **EP 1 027 868 A1**
(43) Veröffentlichungstag der Anmeldung: **16.08.2000**
(21) Anmeldenummer: 99102444.9
(22) Anmeldetag: 09.02.1999
(51) Int. Cl.: A61B 17/80

(54) **Zugapparat zur Korrektur eines Kiefers**

(71) Anmelder: Müller, Paul A., Dr. med., 8034 Zürich (CH)
(72) Erfinder: Müller, Paul A., Dr. med., 8034 Zürich (CH)
(74) Vertreter: Weiss, Peter, Dr.

(57) **Zusammenfassung**

Bei einem Zugapparat zur Korrektur eines Kiefers an einem Schädel, beispielsweise zur Beseitigung eines Überbisses, Kreuzbisses oder Gaumenspaltes, soll ein Grundkörper (1) mit Elementen zum Festlegen am Kiefer (19) versehen und in Korrekturrichtung (x₁) bewegbar sein. Dabei ist der Grundkörper (1) auch abweichend zur Korrekturrichtung (x₁) bewegbar ist.

## Beschreibung

Die Erfindung betrifft einen Zugapparat zur Korrektur eines Kiefers an einem Schädel, beispielsweise zur Beseitigung eines Überbisses, Kreuzbisses oder Gaumenspaltes, wobei ein Grundkörper mit Elementen zum Festlegen am Kiefer versehen und in Korrekturrichtung bewegbar ist.

Ein derartiger Zugapparat ist aus der DE-A- 195 38 323 bekannt. Aufgabe der vorliegenden Erfindung ist es, diesen Zugapparat zu verbessern und ihn variabler auszugestalten.

Zur Lösung dieser Aufgabe führt, dass der Grundkörper auch abweichend zur Korrekturrichtung bewegbar ist.

Es hat sich herausgestellt, dass die Einstellung der Korrekturrichtung von grosser Bedeutung ist. Die Korrekturrichtung sollte dabei nicht nur zu Beginn der Behandlung festgelegt werden, sondern sie sollte auch im Laufe der Behandlungszeit veränderbar sein und einer wechselnden Gegebenheit angepasst werden können. Dies ist nur möglich, wenn der Grundkörper, wie nach der vorliegenden Erfindung, auch abweichend zur Korrekturrichtung bewegbar ist. Das heisst, der Grundkörper und damit die Zugrichtung kann sich ändernden Gegebenheiten angepassen. Dies ist der Grundgedanke der vorliegenden Erfindung und bedeutet einen wesentlichen Vorteil.

Wie diese Bewegbarkeit des Grundkörpers auch abweichend zur Korrekturrichtung bewerkstelligt wird, soll gegenüber diesem grundlegenden Erfindungsgedanken von untergeordneter Bedeutung sein, jedoch wird nachfolgend ein bevorzugtes Ausführungsbeispiel beschrieben:

Schon aus der DE-A- 195 38 323 ist bekannt, dass der Grundkörper ein Verbindungselement aufweist, zu dem der Grundkörper in Korrekturrichtung bewegbar ist und welches eine feste Verbindung zu einer feststehenden Fixation am Kiefer bzw. Schädel selbst aufweist. Durch diese feststehende Fixation erfolgt eine ungefähre grundlegende Festlegung des gesamten Zugappates am Schädel. Das Verbindungselement selbst hält von der feststehenden Fixation in der Regel einen gleichbleibenden Abstand ein, während der Grundkörper relativ zu dem Verbindungselement bewegbar ist. Dies geschieht mittels eines Antriebes, der in der DE-A- 195 38 323 beschrieben ist. Deshalb wird hier auf diese Schrift Bezug genommen und diese auch zum Inhalt der vorliegenden Anmeldung gemacht.

Bevorzugt ist diesem Betätigungselement eine mobile Fixation aufgesetzt, wobei das Aussengewinde des Betätigungselementes auch diese mobile Fixation durchgreift, so dass bei einem Drehen des Betätigungselementes die mobile Fixation entlang dem Gewinde wandert und damit ein Abstand zwischen mobiler Fixation und Grundkörper nicht verändert wird.

Etwa senkrecht zu dem Betätigungselement wird die mobile Fixation von einer Verbindung durchsetzt, die mit einem Ende an der feststehenden Fixation festliegt. Die Verbindung ist so ausgestaltet, dass die mobile Fixation entlang von ihr bewegt werden kann. Der Einfachheit halber ist auch hier wieder ein Gewindeabschnitt vorgesehen, welcher eine entsprechende Bohrung mit Innengewinde in der mobilen Fixation durchsetzt. Bei einem Drehen dieses Gewindeabschnittes kann nun die mobile Fixation entlang diesem Gewindeabschnitt wandern.

Wie oben erwähnt, sind aber auch viele Möglichkeiten einer derartigen Verbindung denkbar und sollen von der vorliegenden Erfindung umfasst sein.

Wesentlicher Vorteil der vorliegenden Erfindung ist, dass mittels der Verbindung zwischen feststehender Fixation und mobiler Fixation eine Zugrichtung eingestellt werden kann, die dann bei Drehung des Betätigungselementes nicht mehr verändert wird. Sie kann erst bei Betätigung der Verbindung selbst wieder verändert werden. Dies kann im Laufe der Behandlung jederzeit geschehen.

Somit kann bei einem Zug, der auf die Maxilla nach vorne wirkt, die Aufklappbewegung der Maxilla nach unten oder oben gesteuert oder auch verhindert werden, und zwar nur mit einem Betätigen der Verbindung zwischen der feststehenden Fixation und der mobilen Fixation.

Weitere Vorteile, Merkmale und Einzelheiten der Erfindung ergeben sich aus der nachfolgenden Beschreibung bevorzugter Ausführungbeispiele sowie anhand der Zeichnung; diese zeigt in ihrer einzigen Figur eine perspektivische Ansicht eines erfindungsgemässen Zugapparates an einer feststehenden Fixation.

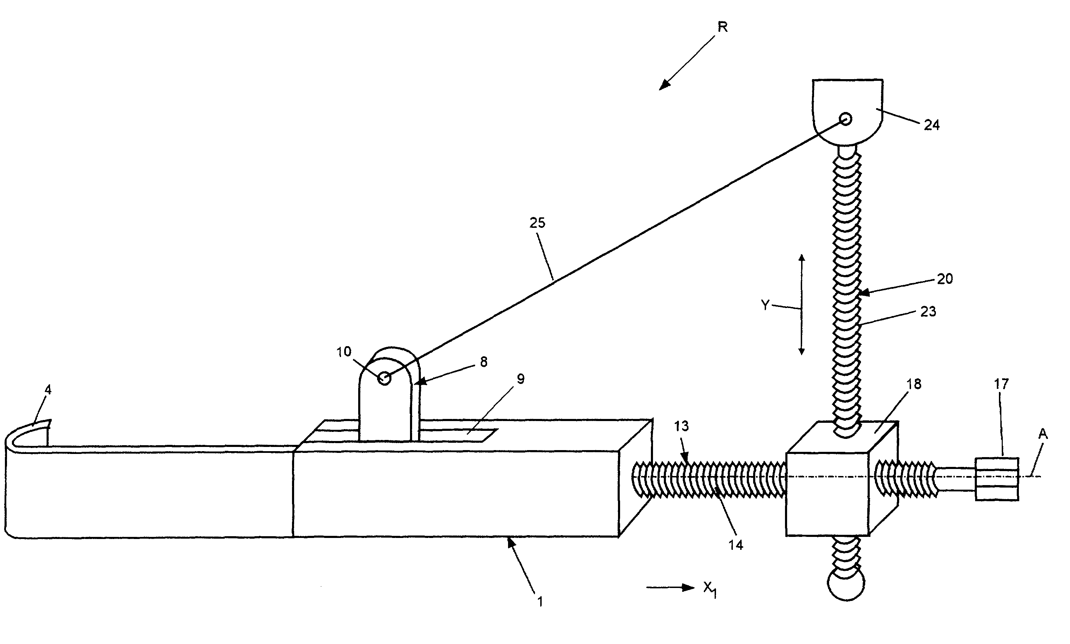

Ein erfindungsgemässer Zugapparat R, dessen Funktion weiter unten beschrieben wird, weist gemäss Figur 1 einen Grundkörper 1 auf. An diesen Grundkörper 1 schliesst ein Haken 4 an. Dieser Haken 4 hintergreift, wie später beschrieben, einen Oberkiefer (Maxilla) einseitig.

In den Grundkörper 1 ist, wie in der DE-A- 195 38 323 beschrieben, eine Durchgangsbohrung eingeformt, in welcher ein Schieber aufgenommen ist, der vor allem entgegengesetzt zur Korrekturrichtung x₁ bewegbar ist.

Einends ist dem Schieber ein Verbindungselement 8 aufgesetzt, welches aus dem Grundkörper 1 bzw. einem Schlitz 9 herausragt. Dieses Verbindungselement 8 besitzt ein Loch 10, welches der Aufnahme eines später beschriebenen Halteelementes dient.

Andernends des Verbindungselementes 8 weist der Schieber eine stirnwärtige Sacklochbohrung auf, in die ein Zapfen eingesetzt ist. Der Zapfen dreht frei in der Sacklochbohrung und ist Bestandteil eines Betätigungselementes 13, mit dem der Schieber bewegt wird. Hierzu weist das Betätigungselement 13 einen Gewindeabschnitt 14 mit einem Aussengewinde auf, welches ein nicht näher gezeigtes Innengewinde in der Durchgangsbohrung kämmt.

An den Gewindeabschnitt 14 schliesst ein Stangenabschnitt 16 an, der mit einem Sechskant 17 endet. Wird dieser Sechskant 17 von einem entsprechenden Werkzeug angegriffen, so kann das Betätigungselement 13 um seine Längsachse A gedreht werden, wobei es gegenüber dem Schieber frei dreht. Über das Aussengewinde schraubt sich jedoch das Betätigungselement 13 weiter in die Durchgangsbohrung ein, so dass es den Schieber entgegen der Korrekturrichtung x₁ bewegt, was eine Bewegung des Grundkörpers 1 in Korrekturrichtung x₁ zur Folge hat.

Auf dem Betätigungselement 13 befindet sich eine mobile Fixation 18, welche entlang dem Betätigungselement 13 bewegbar ist. Diese mobile Fixation 18 wird von einer Verbindung 20 durchsetzt, die eine Verbindung mit einer feststehenden Fixation 24 herstellt. Bei dieser feststehenden Fixation 24 kann es sich beispielsweise um ein Titanplättchen handeln, welches, wie in der DE-A- 195 38 323 erwähnt, an einem anderen Teil des Oberkiefers bzw. des Schädels festgelegt wird. Wesentlich ist, dass durch diese Verbindung 20 die mobile Fixation 18 in Richtung y gegenüber der Fixation 24 bewegt werden kann.

Im gezeigten Ausführungsbeispiel weist die Verbindung 20 ein Aussengewinde 23 auf. Auf diesem Aussengewinde 23 läuft die mobile Fixation 18.

Die Funktionsweise der vorliegenden Erfindung ist folgende:

Wie in der DE-A- 195 38 323.0 beschrieben, wird der Zugapparat R beispielsweise an einen Oberkiefer eines menschlichen Schädels so angelegt, dass der Haken 4 den Oberkiefer hinter dem letzten Backenzahn hintergreift. Das Verbindungselement 8 wird beispielsweise über einen Draht 25 mit der feststehenden Fixation 24 am Kiefer oder Schädel verbunden. Nunmehr erfolgt eine Einstellung der Zugrichtung, das heisst, in der Regel eine Einstellung der Korrekturrichtung durch die Verbindung 20, da mit dieser Verbindung 20 die Lage des Grundkörpers 1 exakt definiert werden kann.

Um jetzt einen Zug auf die Maxilla auszuüben, wird das Betätigungselement 13 gedreht, wie dies in der DE-A- 195 38 323 beschrieben ist. Hierdurch wird das Verbindungselement 8 entgegen der Korrekturrichtung x₁ und somit der Grundkörper 1 und damit auch der Haken 4 in Korrekturrichtung verschoben. Gleichzeitig wandert aber auch die mobile Fixation entgegen der Korrekturrichtung x₁, so dass die einmal eingestellte Zugrichtung auch bei weiterer Drehung des Betätigungselementes 13 nicht verändert wird.

| Positionszahlenliste | | | | | |
|---|---|---|---|---|---|
| 1 | Grundkörper | 34 | | 67 | |
| 2 | | 35 | | 68 | |
| 3 | | 36 | | 69 | |
| 4 | Haken | 37 | | 70 | |
| 5 | | 38 | | 71 | |
| 6 | | 39 | | 72 | |
| 7 | | 40 | | 73 | |
| 8 | Verbindungselement | 41 | | 74 | |
| 9 | Schlitz | 42 | | 75 | |
| 10 | Loch | 43 | | 76 | |
| 11 | | 44 | | 77 | |
| 12 | | 45 | | 78 | |
| 13 | Betätigungselement | 46 | | 79 | |
| 14 | Gewindeabschnitt | 47 | | | |
| 15 | | 48 | | | |
| 16 | Stangenabschnitt | 49 | | A | Längsachse |
| 17 | Sechskant | 50 | | | |
| 18 | mobile Fixation | 51 | | | |
| 19 | | 52 | | | |
| 20 | Verbindung | 53 | | | |
| 21 | Teil | 54 | | R | Zugapparat |
| 22 | Teil | 55 | | | |
| 23 | Aussengwinde | 56 | | | |
| 24 | feststehende Fixation | 57 | | | |
| 25 | Draht | 58 | | Y | Richtung |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Zugapparat zur Korrektur eines Kiefers an einem Schädel, beispielsweise zur Beseitigung eines Überbisses, Kreuzbisses oder Gaumenspaltes, wobei ein Grundkörper (1) mit Elementen zum Festlegen am Kiefer (19) versehen und in Korrekturrichtung (x₁) bewegbar ist,
dadurch gekennzeichnet,
dass der Grundkörper (1) auch abweichend zur Korrekturrichtung (x₁) bewegbar ist.

2. Zugapparat nach Anspruch 1, dadurch gekennzeichnet, dass der Grundkörper (1) ein Verbindungselement (8) aufweist, im Verhältnis zu dem der Grundkörper (1) in Korrekturrichtung (x₁) bewegbar ist und welches eine feste Verbindung zu einer feststehenden Fixation (24) aufweist.

3. Zugapparat nach Anspruch 1 oder 2, dadurch gekennzeichnet, dass der Fixation (24) eine Verbindung (20) zugeordnet ist, an der entlang der Grundkörper (1) oder ein anderes dem Grundkörper (1) zugeordnetes Element (13) bewegbar ist.

4. Zugapparat nach Anspruch 3, dadurch gekennzeichnet, dass dem Grundkörper (1) ein Betätigungselement (13) zugeordnet ist, auf dem eine mobile Fixation (18) aufsitzt, welche auch von der Verbindung (20) mit der feststehenden Fixation (24) durchsetzt ist.

5. Zugapparat nach Anspruch 4, dadurch gekennzeichnet, dass das Betätigungselement (13) einen Gewindeabschnitt (14) aufweist, entlang dem die mobile Fixation (18) bewegbar ist.

6. Zugapparat nach einem der Ansprüche 3 - 5, dadurch gekennzeichnet, dass die Verbindung (20) ein Aussengewinde (23) aufweist, auf welchem sich die mobile Fixation (18) bewegt.
